# EUROPEAN PATENT SPECIFICATION

(11) **EP 0 347 933 B1**
(45) Date of publication and mention of the grant of the patent: **14.09.1994**
(21) Application number: 89111471.2
(22) Date of filing: 23.06.1989
(51) Int. Cl.: C12Q 1/56, G01N 33/86

(54) **Assay for soluble crosslinked fibrin polymers**
Test für lösliche quervernetzte Fibrinpolymere
Essai pour les polymères réticulés de fibrine

(30) Priority: 24.06.1988 US 213572
(43) Date of publication of application: 27.12.1989
(73) Proprietor: RESEARCH CORPORATION TECHNOLOGIES, INC., Tucson Arizona 85711 (US)
(72) Inventor: Marder, Victor J., Rochester, NY (US); Francis, Charles W., Rochester, NY (US)
(74) Representative: Hansen, Bernd, Dr. Dipl.-Chem.

(56) References cited:
- EP-A- 0 122 478
- EP-A- 0 173 916
- WO-A-88/01514
- BIOCHEMISTRY, vol. 18, no. 6, 1979, American ChemicalSociety, Easton, PA (US); S.A. OLEXA et al., pp. 991-995#
- THROMBOSIS RESEARCH, vol. 31, 1983; pp. 767-778#

## Description

The present invention relates to an assay for use in the diagnosis of patients at risk of thrombosis and in monitoring the effects of treatment of such patients. More particularly, the present invention relates to a method for measuring the amount of soluble crosslinked fibrin polymers involving the in vitro treatment of patient sample, e.g. plasma, with a plasminogen activator such as tissue-plasminogen activator (t-PA) or active plasmin to generate D-dimer products of soluble fibrin polymers, the amount of D-dimer generated being directly proportional to the amount of soluble crosslinked fibrin polymers in the patient sera, the amount being indicative of thrombin and factor XIII activation, i.e. on-going thrombosis, that may indicate a hypercoagulable state.

Much effort has been expended in recent years to develop methods for measuring activation products of fibrinogen. The reason is that fibrinogen to fibrin conversion may be involved in many different pathological conditions, including the prethrombotic state and disseminated intravascular coagulation. Nevertheless, the presently available methods for direct determination of circulating fibrin (soluble fibrin) are not practical since they are either difficult to perform or suffer lack of sensitivity and of specificity. Such methods have consisted, among others, of chemical purification and chromatographic assay of soluble fibrin in human plasma or by a serial dilution protamine sulfate testing.

In theory, fibrin formation can be detected indirectly by determination of released fibrinopeptides. However, apart from being difficult to analyze, the fibrinopeptides have short half-lives and may not give the same information as direct determination of soluble fibrin.

It has also been proposed to use a method based on the stimulatory effect of fibrin obtained on the t-PA-catalyzed activation of plasminogen (Wiman et al., Thombosis and Haemostatis 1986, 55: (2) 189). In this assay the t-PA activity increases in the presence of soluble fibrin and can be back-calculated to indicate soluble fibrin concentration. The end point determination, however, is one of enzymatic activity by t-PA on a plasmin-sensitive chromogenic substrate rather than a degradation fragment of the fibrin.

It has also been proposed to use immunoassays that utilize monoclonal antibodies raised against the unique antigenic determinants of crosslinked fibrin to specifically measure the degradation products of crosslinked fibrin without being influenced by the degradation products of fibrinogen (Rylatt et al. Thomb. Res. 1983, 31:767-78) (Whitaker et al., J. Clin. Pathol. 1984; 37:882-7) (Gaffney et al., Thrombosis and Haemostasis 1987; 58-231, abstract). The D-dimer is one of the degradation products of crosslinked fibrin which is stable and resists further digestion by plasmin. Elevated serum or plasma levels of D-dimer have been found in patients with pathologic fibrinolysis and after streptokinase-induced thrombolysis in patients with deep venous thrombosis or pulmonary embolus (Lew et al., J.A.C.C., 1986, Vol. 1, No. 6, 1320-4). Therefore, this technique is potentially useful only for monitoring the end-product of fibrinolysis, for example, during physiologic thrombolysis or with therapy using plasminogen activators.

It would be highly desirable, therefore, to provide a rapid and simple assay that can be used for the in vitro measurement of soluble fibrin concentration, for example, in patient plasma as a useful reflection of the so-called "hypercoagulability state" or pre-thrombotic state reflecting an increased tendency to form fibrin. The assay should be for routine laboratory use and for the determination of large numbers of samples and with great accuracy.

A diagnostic immunoassay for measuring the increased reactivity of a test material for fragment D-dimer produced by the addition of a plasminogen activator to the sample, such as plasma, can be used to determine the original concentration of crosslinked fibrin polymer in the patient sample. Thus, the plasma concentration of D-dimer following degradation of soluble fibrin to D-dimer in vitro could be used as a simple indirect measure of circulating crosslinked fibrin polymer in the in vivo condition.

Subject matter of the present invention is a process according to claim 1 for the in vitro determination of crosslinked soluble fibrin polymers in a patient sample which comprises contacting said sample with a proteolytic enzyme to generate the D-dimer fragment of said fibrin polymers and measuring the amount of said D-dimer.

Further subject matter is a process according to claim 3 for the in vitro determination of crosslinked soluble fibrin polymers in a patient sample which comprises contacting said sample with a proteolytic enzyme to generate the D-dimer fragment of said fibrin polymers and measuring the amount of said D-dimer by the use of an immunoassay specific to said D-dimer.

Subject matter of the present invention is also a diagnostic kit according to claim 10 for assaying the amount of D-dimer in a patient sample comprising a carrier being compartmented to receive a series of containers in close confinement which comprises:
(a) a first container containing a measured amount of standard containing crosslinked soluble fibrin polymers,
(b) a second container containing a measured amount of a proteolytic enzyme capable of generating the D-dimer fragment of said fibrin polymers, and
(c) a third container containing an antibody specific to said D-dimer.

Preferred embodiments of the process and kit of the invention are subject matter of claims 2 and 4 to 9, and of claims 11 to 13, respectively.

The present invention provides a novel diagnostic assay having the foregoing advantages which involves the in vitro treatment of the patient sample with a plasminogen activator (such as t-PA) or active plasmin, to generate D-dimer. The results of this test could provide insight into and greatly facilitate the evaluation of patients with thrombosis or at risk of thrombosis and be highly useful in monitoring the effects of treatment of such patients. The amount of D-dimer formed is determined by an immunoassay specific for D-dimer. The amount of D-dimer generated is directly proportional to the soluble fibrin polymers present in patient samples which amount is indicative of thrombin and factor XIII activation, i.e. on-going thrombosis and may indicate a hypercoaguable state.

The present invention thus provides a means for practical determinations of patient samples in short time periods, is applicable for routine laboratory use and for the determination of large numbers of samples and with great accuracy. The materials needed are simple, easily transportable, applicable at large and small centers and even in doctor's offices and amenable to large scale automation. The use of crosslinked fibrin polymers as markers of hypercoagulability affords a very practical approach in that it deals with the final pathway toward clot formation, rather than dealing with intermediate steps in a complex coagulation pathway. Thus, the present invention provides an in vitro degradation to D-dimer that can be used as a simple indirect measurement of plasma crosslinked fibrin polymer concentration in patient samples. Such samples include but are not limited to blood, serum, plasma, urine, cerebrospinal fluid, ascites, exudate and transudate and in vitro fractions of such materials. The test can be useful for evaluating pre-thrombotic disorders which are associated with development of thrombi, and thrombotic disorders such as myocardial infarction, pulmonary embolism and deep vein thrombosis. Other pathologic conditions associated with fibrin formation, such as neoplasm, inflammatory and immunologic disorders, as well as the post-operative state and other trauma, and other miscellaneous pathologic states, may also be associated with elevated levels of crosslinked fibrin.

Figure 1 shows the crosslinked fibrin polymers in plasma following addition of thrombin.

Figure 2 shows the quantitation of increase in crosslinked fibrin polymers in plasma after thrombin addition relative to clotting time.

Figure 3A shows a t-PA-induced degradation of fibrinogen and crosslinked fibrin polymers in plasma.

Figure 3B is a comparison of rates of plasmic degradation of fibrin dimer and fibrinogen/fibrin monomer.

Figure 4 shows plasma immunoreactivity in a D-dimer ELISA assay before and after treatment with thrombin and/or t-PA.

Figure 5 shows the changes in plasma fibrinogen after incubation with thrombin and/or t-PA.

The present invention is based upon the discovery that it is possible to provide an effective assay method to measure the amount of a proteolytic derivative, specifically fragment D-dimer (DD), generated by the degradation of soluble crosslinked fibrin polymers following the addition of a plasminogen activator, for example t-PA to a patient sample, e.g. plasma, in vitro. The degradation of crosslinked fibrin polymers to proteolytic derivatives induces molecular change that can be exploited with simple detection technique against fragment DD which is a well defined molecular entity that can be measured by various immunologic technique using a specific antibody. Crosslinked fibrin polymers react little if at all in these immunologic systems and, therefore, the increased reactivity of a test material for fragment DD, after t-PA treatment, for example, of the material, bears a direct relationship to the original concentration of the crosslinked fibrin polymer in patient sample. Thus a quantitation of the original concentration of circulating crosslinked fibrin polymers in patient sample is achieved by an in vitro test which facilitates the diagnosis of patients at risk of thrombosis.

Under physiological conditions, fibrin formation is localized either intravascularly in the formation of hemostatic plugs or extravascularly in inflammatory foci. However, low concentrations of soluble fibrin circulate, and increased concentrations have been identified in patients with thrombotic disease reflecting a systemic effect of thrombin activity that can also be measured by plasma fibrinopeptide levels. Soluble fibrin also facilitates the thrombin cleavage of factor XIII to a transglutaminase which can form intermolecular covalent crosslinks between γ chain pairs of adjacent fibrin monomers. We have recently demonstrated (Francis et al., Circulation 1987; 75:1170-1177) a low concentration off γ chain crosslinked fibrin dimer in plasma of normal individuals and a significant increase in patients presented with acute myocardial infarction.

Pharmacologic stimulation of fibrinolysis in the treatment of thrombotic disease results in a variable degree of degradation of circulating fibrinogen in addition to the desired effect of thrombolysis. Because the γ chain isopeptide crosslink in stabilized fibrin renders this portion of the molecule resistant to plasmic degradation, plasmic degradation products of crosslinked fibrin differ structurally from those of fibrinogen. As stated hereinbefore this difference has been exploited in the development of several methods to measure circulating crosslinked fibrin-specific degradation products as potential markers of clot lysis. The present invention utilizes antibodies specific for crosslinked fibrin degradation products in quantitative immunologic assays.

An antibody, which exhibits the same immunological properties as those of that monoclonal antibody which is secreted by hybridoma and DD-3B6/22 (Rylatt et al. and Whitaker et al., supra) that is directed toward an epitope near the γ crosslink site present in crosslinked fibrin degradation products such as fragment DD is preferably used in the present process, but other antibodies reactive preferentially with D-dimer or other degradation products rather than with fibrin could also be used. Since crosslinked fibrin polymers and crosslinked fibrin degradation products both contain the γγ crosslink, the possibility exists that both would react with this antibody. However, this potential problem is not the case, as demonstrated below (see experiment). Crosslinked fibrin polymer formation is induces in vitro by exposure of plasma to low concentrations of thrombin; the reactivity of soluble fibrin polymers is assessed in an enzyme-linked immunosorbent assay (ELISA) using a monoclonal antibody reactive with the γγ chain crosslink. In addition, t-PA-treated samples of plasma and thrombin-exposed plasma, in which fibrin is converted to D-dimer, are assessed for reactivity in the same immunoabsorbent assay with antibody against γγ chain crosslink. The changes in immunoreactivity with thrombin-induced formation of fibrin polymers and with t-PA-induced degradation have important implications for interpreting studies of fibrin and fibrinogen degradation products during thrombolytic therapy and for the development of assays to detect plasma crosslinked fibrin polymers.

In the further detailed description and example which follow, it should be noted that the following materials and methods were used:
Proteins. Human fibrinogen (grade L) was purchased from Helena Laboratories (Beaumont, TX) and was 93% clottable. Fibrinogen concentration was determined by measurement of optical density at 280 nm using an extinction coefficient of 15.1. Human thrombin (3200 NIH U/mg) was purchased from Calbiochem-Behring Corp. (La Jolla, CA), hirudin and bovin serum albumin were from Sigma Chemical Co. (St. Louis, MO), aprotinin from Mobay Chemical Co. (New York, NY), and tissue plasminogen activator (100,000 IU/mg) from Burroughs Wellcome Co. (Research Triangle Park, NC). Goat anti-human fibrinogen IgG was obtained from Cappel Laboratories (Cochranville, PA). Purified factor XIII concentrate (Fibrogammin), prepared from human placenta, was provided by Behringwerke/Hoechst-Roussel (Somerville, NJ). Factor XIII was assayed by dansyl cadaverine incorporation into casein and the activity expressed in relation to pooled normal plasma defined as 100%.

Radiolabeling. Fibrinogen was labeled with the iodogen technique to a specific activity of 0.05 mCi/mg using 0.02 mg iodogen and 100 uCi ¹²⁵I per ml of fibrinogen (20 mg/ml). Labeled fibrinogen was 93% clottable and showed the same electrophoretic mobility on SDS, 7% polyacrylamide gel electrophoresis after disulfide bond reduction. Goat anti-human fibrinogen IgG was radioiodinated to a specific activity of 0.4 mCi/mg using the lactroperoxidase method (Marchalonis, J.J., Biochem J., 1969; 113:299). Bound and free iodine were separated after labeling by chromatography on Sephadex® G-25 (Pharmacia Fine Chemicals, Piscataway, NJ).

Preparation of fibrin polymers. Fibrinogen was dissolved to a final concentration of 10 mg/ml, calcium chloride added to a final-concentration of 0.025 mol/L and factor XIII to a concentration of 12 units/ml. Thrombin was then added at a final concentration of 0.0025 units/ml, the solution was incubated at 37°C for intervals of 10 to 45 min, and the thrombin activity was inhibited by addition of hirudin to a final concentration of two units/ml.

Blood Samples. After obtaining informed consent, blood was collected from normal volunteers into sodium citrate (0.4% final concentration) and plasma separated from red cells after centrifugation at 3500 g for 15 min at 4°C.

Electrophoretic Analysis. Fibrinogen and fibrin derivatives were identified in plasma after SDS 2% agarose electrophoresis (Connaghan, D.G. 1985; Blood 65:5891). Plasma was prepared for electrophoresis by diluting 1:20 in 0.01M phosphate buffer, pH 7, containing 1.7% SDS and heating at 100°C for 5 min or 60°C for one hour. Samples of 10 µl were electrophoresed at 150 volts on a flat bed electrophoretic apparatus (Pharmacia Fine Chemicals, Piscataway, NJ) with cooling to 10°C. Electrophoresis was allowed to proceed until the tracking dye had migrated 10 cm. Gel scanning was performed with a Quik-Scan Jr. (Helena Laboratories, Beaumont, TX).

Western blotting was performed using a modification of the method of Towbin and colleagues (Proc. Nat'l. Acad. Sci. USA 76:4350, 1979), using a Transblot Cell (BioRad Laboratories, Richmond, CA). Transfer to nitrocellulose paper (pore size 0.2 µm, Schleicher and Schuell, Keene, NJ) was done in 0.01M Tris hydrochloric buffer, pH 8.3 containing 0.096M glycine and 20% (v/v) methanol at 60 volts for 3 h at 20°C. The nitrocellulose paper was incubated at 3°C for 24 h in 0.05M phosphate buffer, pH 7.3, containing 0.15M sodium chloride and 3% (w/v) bovine serum albumin. This was removed, and the paper was incubated for 2 h at 20°C with gentle rocking with 50 ml of 0.05M phosphate buffer, pH 7.3, containing 0.15M sodium chloride, 3% (w/v) bovine serum albumin and 0.05% (v/v) Tween® 20 and 2 x 10⁻⁵ Ci ¹²⁵I-labeled goat anti-human fibrinogen IgG. The nitrocellulose paper was then washed with 6 volumes of 40 ml each of 0.05M phosphate buffer, pH 7.5, containing 0.15M sodium chloride and 0.05 (v/v) Tween® 20 using gentle agitation on a platform shaker for 10 min for each wash. Following drying, the paper was processed for autoradiography using Kodak® X-Omat film (Eastman Kodak, Rochester, NY) with exposure times up to 72 h.

ELISA. Crosslinked fibrin degradation products were measured with an ELISA (Dimer Test, American Diagnostica, Greenwich, CT) employing a monoclonal antibody which exhibits the same immunological properties as those of that monoclonal antibody which is secreted by hybridoma DD/3B6/22 reactive with a site near the factor XIIIₐ mediated crosslink (Rylatt et al. and Whitaker et al., supra). Precoated plates were used and results calculated using a standard curve prepared with purified fragment DD provided by the manufacturer at concentrations from 40 ng/ml to 5000 ng/ml. Plasma was assayed undiluted prior to incubation with t-PA and at 1:5 dilution after incubation.

The invention will be described in greater detail in conjunction with the following specific example.

### EXAMPLE

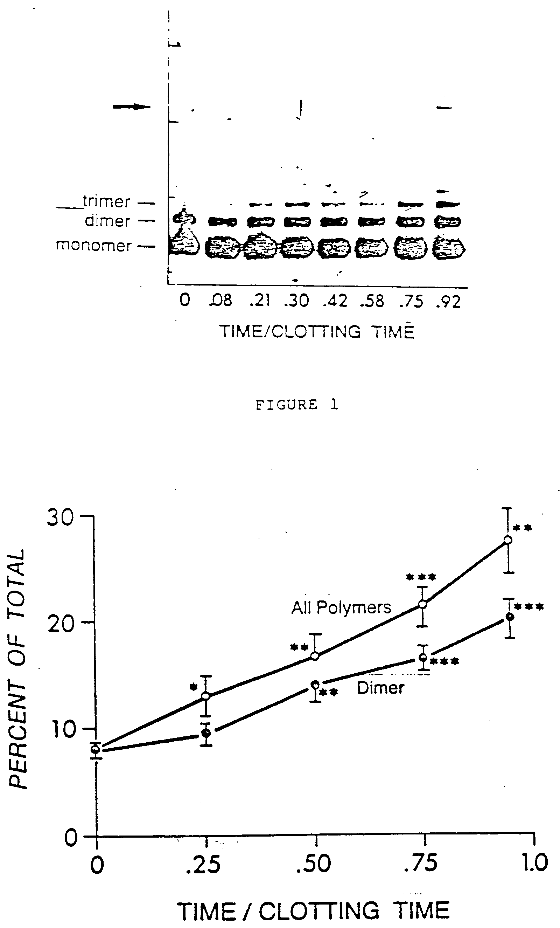

The addition of a low concentration of thrombin to normal plasma (0.025 U/ml final concentration) results in a progressive increase in crosslinked fibrin polymers before clot formation, as shown in the autoradiogram (Figure 1). Prior to thrombin addition (time 0), bands corresponding to fibrin dimer and a trace of trimer are present in the ¹²⁵I radiolabeled fibrinogen. After thrombin addition, but prior to clot formation, the dimer band becomes more prominent and larger polymeric forms are seen. The greatest change occurs in the last sample prior to clotting (at 0.92 clotting time), when up to six polymeric forms can be seen and some protein fails to enter the 2% agarose gel.

The formation of crosslinked fibrin polymers in plasma following addition of thrombin is shown in Figure 1. Pooled normal citrated plasma containing ¹²⁵I radiolabeled fibrinogen is incubated with thrombin (0.025 U/ml final concentration) and calcium chloride (10 mM final concentration). At intervals, aliquots are withdrawn and prepared for electrophoresis by diluting 1/20 is SDS containing diluent and heating at 60°C for one hour. Samples of 10 µl are electrophoresed and the autoradiogram prepared following drying with an exposure time of 3 days. Time/clotting time indicates the time at which the sample is withdrawn divided by the observed clotting time. The arrow indicates the location of application wells.

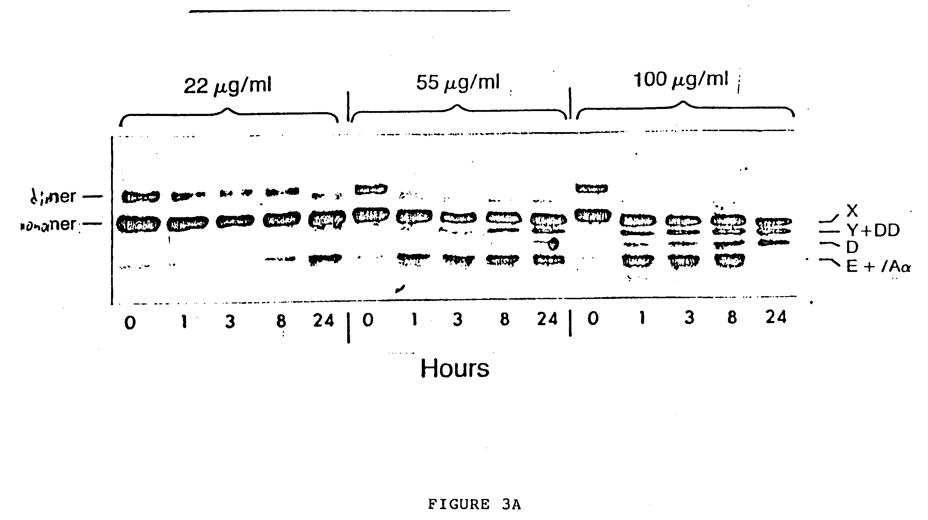

The use of ¹²⁵I radiolabeled fibrinogen allows accurate quantitation of fibrin polymer formation by slicing the fixed gel after staining and electrophoresis and then counting individual slices to determine the proportion of each polymer band (Figure 2). Prior to the addition of thrombin, 8% of the radiolabel migrates as crosslinked dimer and 8.2% as dimer plus larger polymers. The proportion of crosslinked polymer increases progressively following addition of thrombin, with a peak of 20% dimer and 27% total crosslinked polymer present just prior to visible fibrin formation. A statistically significant increase in dimer concentration occurs by 0.5 clotting time and in total crosslinked polymer concentration at 0.25 clotting time. Polymeric forms larger than dimer are most abundant immediately prior to visible clot formation at 0.9 clotting time when they represent 7% of the total. No increase in polymer formation is seen when the same experiment is performed using plasma anticoagulated with EDTA (.1% final concentration) and without added calcium chloride.

Figure 2 shows in detail the quantitation of increase in crosslinked fibrin polymers in plasma after thrombin addition relative to clotting time. Thrombin (0.025 to 0.1 u/ml, final) and calcium chloride (10 mM final) are added to pooled normal plasma containing ¹²⁵I radiolabeled fibrin. Aliquots are withdrawn at intervals prior to clot formation during incubation at 37°C and prepared for electrophoresis. Following staining with Coomassie Blue, bands corresponding to monomer, dimer and larger polymers are cut from the gel and counted. Time/clotting time indicates the time of withdrawal of the sample divided by the final clotting time. Statistically significant increases in comparison with time 0 values are indicated: *, p <0.05; **, p <0.01; ***, p <0.005.

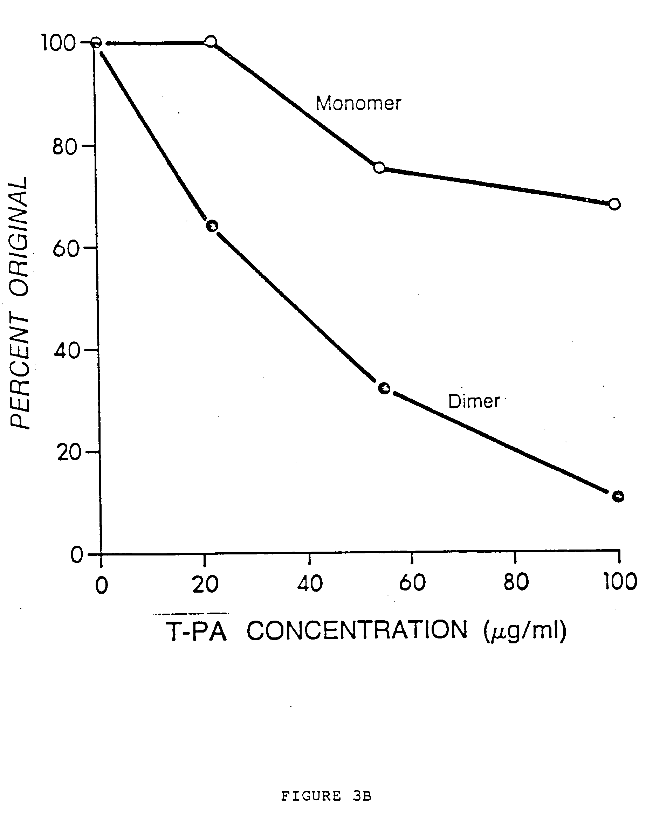

Plasmic degradation of soluble crosslinked fibrin polymers is followed electrophoretically after addition of t-PA to normal plasma to which a preparation of ¹²⁵I radiolabeled fibrin polymer is added (Figure 3). At 16 µg/ml t-PA the fibrinogen band does not change, but the dimer band is fainter at 24 h and migrates further, consistent with degradation to fragment XX. At 55 and 100 µg/ml t-PA, fibrinogen is degraded to fragment X within the first three hours but then persists during longer incubations, showing little cleavage to smaller fragment Y, D and E. In contrast to the plasmic resistance of fibrinogen and fragment X, the degradation of the crosslinked fibrin dimer occurs rapidly. At 55 µg/ml and 100 µg/ml t-PA, most fibrin dimer is degraded by one hour with little dimer or fragments larger than fibrinogen remaining after longer incubations. The rates of degradation of fibrin dimer and fibrinogen are compared following gel scanning, and at all concentrations of t-PA tested, degradation of the dimer is more rapid than the monomer (Figure 3B). At 16 µg/ml t-PA, there is no decrease in the monomer at 3 h while the dimer has decreased to 64% of original. The difference in degradation between monomer and dimer is greater at higher enzyme concentrations with only 10% of dimer remaining at 3 h at 100 µg/ml t-PA compared to 68% of monomer.

t-PA induced degradation of fibrinogen and crosslinked fibrin polymers are shown in Figures 3A and 3B. Tissue plasminogen activator at concentrations of 16 µg/ml, 55 µg/ml, and 100 µg/ml are added to pooled normal plasma containing an ¹²⁵I radiolabeled preparation of crosslinked fibrin polymers. During incubation at 37°C, aliquots are withdrawn at the indicated times and prepared for electrophoresis. Following staining and drying, the autoradiogram is exposed for six days. The 2% agarose gel system has limited resolution of fragments smaller than fibrinogen; fragments Y and DD overlap and cannot be resolved and Fragment E migrates with degradation products of the A chain.

Figure 3B shows a comparison of rates of plasmic degradation of fibrin dimer and fibrinogen/fibrin monomer. The lanes in Figure 3A corresponding to the three hour t-PA incubation are scanned, and the rate or degradation of fibrin dimer and fibrinogen/fibrin monomer are calculated in comparison with the time 0 sample. Since the gel system does not distinguish between fibrinogen, fibrin monomer and fragment X, these are measured together, and the decrease shown represents coiled-coil cleavage to smaller fragments Y, D, or E. Similarly, since plasmic derivatives smaller than fibrin dimer but larger than fibrinogen are difficult to measure separately using the gel scanning technique, the value for fibrin dimer represent all bands larger than fibrinogen, thereby tending to overestimate the remaining fibrin dimer.

**TABLE 1**

| Concentration of D-dimer by ELISA in pooled normal plasma after one hour incubation with t-PA (mean of 2 experiments) | |
|---|---|
| t-PA concentration µg/ml | Plasma D-dimer concentration ng/ml |
| 0.001 | 100 |
| 0.01 | 325 |
| 0.1 | 350 |
| 1 | 500 |
| 10 | 1000 |
| 100 | 2700 |
| 400 | 2700 |

Since plasma crosslinked fibrin polymers increase after thrombin exposure, and plasmic degradation of polymers produces fragments DD, fragment DD is quantitated following incubation of plasma with t-PA before and after thrombin treatment to determine the effect of increased fibrin polymers on the apparent D-dimer concentration, before and after plasmic degradation by t-PA. The plasma D-dimer reactivity increases after in vitro treatment with t-PA for one hour in a concentration-dependent manner, reaching a plateau above 100 µg/ml of t-PA (Table 1).

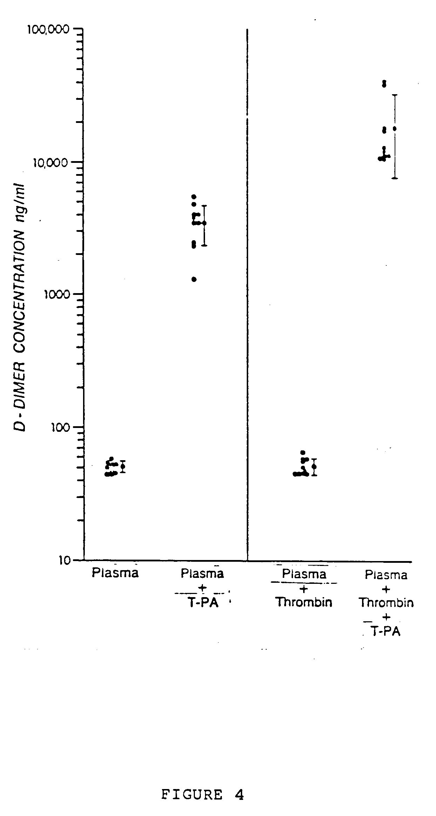

The mean D-dimer concentration in normal citrated plasma is 50.3 ± 4.5 ng/ml, with no change after incubation with thrombin (0.05 units/ml final concentration) for 20 min at 25°C (Figure 4). The DD concentration increases markedly to a mean of 3,560 ± 1235 ng/ml after incubation with t-PA (200 µg/ml). Following exposure to thrombin, t-PA incubation produces an even larger increase in D-dimer concentration to a mean of 18,580 ± 11,780 ng/ml. An increase in the DD concentration following t-PA digestion occurs in all plasma samples with no overlap between those digested before and after thrombin treatment.

Figure 4 shows plasma immunoreactivity in a D-dimer ELISA assay before and after treatment with thrombin and/or t-PA. D-dimer concentration in citrated plasma collected from 10 normal individuals is 50.3 ± 4.5 ng/ml (mean ± SD) (left); after incubation with t-PA (200 µg/ml, final concentration) for one hour at 37°C, the concentration is 3,560 ± 1,238 ng/ml. The same plasmas are also incubated with thrombin 0.05 u/ml final concentration) for 20 min at 37°C. Prior to incubation with t-PA, there is no change in D-dimer reactivity (51.5 ± 7.5) ng/ml, but values for all samples increase markedly following t-PA incubation to a mean of 18,580 ± 11,780 ng/ml.

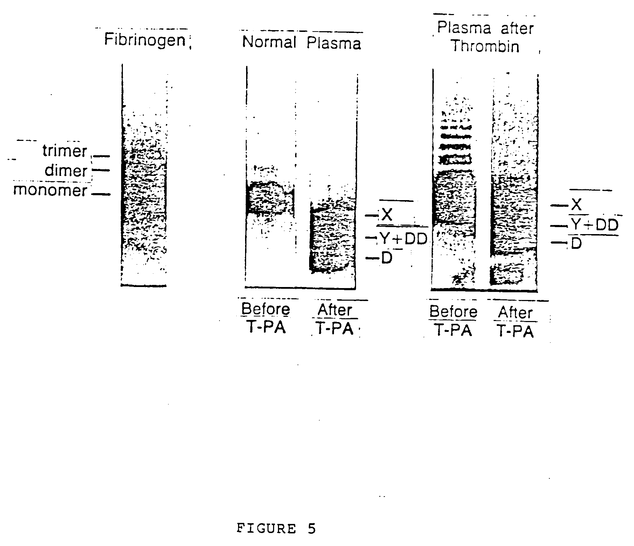

The changes after thrombin and t-PA are reflected in the gel pattern seen after Western blotting with antifibrinogen antiserum (Figure 5). Normal plasma shows a heavy fibrinogen and faint dimer band; after thrombin exposure, more dimer is seen and bands corresponding to five larger crosslinked polymers are present. Following incubation with t-PA, bands corresponding to polymers are absent, confirming complete degradation, and only fragments smaller than fibrinogen are present.

Figure 5 shows changes in plasma fibrinogen after incubation with thrombin and/or t-PA. A single normal plasma sample is treated as in Figure 4 with thrombin (0.05 U/ml for 20 min at 37°C), t-PA (200 µg/ml for 1 h at 37°C) or thrombin followed by t-PA. Following SDS 2% agarose gel electrophoresis, Western blotting is performed using an antifibrinogen antiserum.

The foregoing experiments demonstrate several effects on plasma fibrinogen resulting from exposure to a low concentration of thrombin and/or activation of fibrinolysis by t-PA. First, there is a progressive and significant increase in soluble crosslinked fibrin polymers prior to clot formation in vitro (See Figures 1 and 2) resulting from the combined action of thrombin and actor XIII. Second, fibrin polymers are degraded more rapidly than fibrinogen (Figure 3), indicating that the fibrin specificity of t-PA, demonstrated with insoluble fibrin, is retained by the soluble crosslinked fibrin polymers. Third, a monoclonal antibody which exhibits the same immunological properties as those of that monoclonal antibody which is secreted by hybridoma DD/3B6/22, reactive with the γ chain crosslinked epitope in fragment DD, does not react with the same crosslinked γ chain site in fibrin polymers as indicated by the same reactivity of plasma before or following thrombin addition (Figure 4). This indicates either that the crosslink site is obstructed from the antibody or that additional confirmational features associated with degradation are critical in determining antibody reactivity with DD/3B6. Fourth, degradation products of plasma soluble fibrin are recognized by the antibody (Figures 4 and 5); therefore, fragment DD present in blood may derive from such polymers as well as from dissolved clot.

Using several techniques, low concentrations of soluble fibrin have been found in normal plasma, with increased amounts in thrombotic disease. Plasma fibrinopeptide A concentration, elevated in thrombotic diseases, reflects the action of thrombin in cleaving fibrinopeptide A from fibrinogen and is a sensitive marker of systemic thrombin action but an indirect measure of plasma soluble fibrin. A concentration of soluble fibrin in normal plasma of 0.6 to 0.7 mg/dl and a 10-fold increase after acute myocardial infarction has been found using fibrinogen affinity chromatography to purify fibrin molecules containing the gly-pro-arg site exposed by thrombin cleavage. An increase in soluble fibrin in patients with stroke or myocardial infarction has been infered by an increase in large early eluting fibrinogen antigen after gel filtration chromatography.

The crosslinked fibrin polymers identified in Figures 1 and 2 require the additional action of factor XIIIₐ to crosslink the γ chains of fibrin monomers. Several factors support the concept of such coordinated action of thrombin and factor XIIIₐ during activation of coagulation. During spontaneous clotting of plasma in vitro, cleavage of fibrinopeptides and activation of factor XIII are closely related events. The binding of both thrombin and factor XIII to fibrin may explain the enhancement of thrombin-catalyzed factor XIIIₐ formation by fibrin polymers in plasma. Prior studies have provided indirect evidence for circulating crosslinked fibrin polymers by identifying γ chain dimers in extracts of plasma obtained from patients with thrombotic disease. Using an electrophoretic technique we have demonstrated directly a low concentration of crosslinked fibrin dimer in normal plasma and a significant elevation in patients with acute myocardial infarction.

Tissue plasminogen activator has a high affinity for fibrin and preferentially activates fibrin-bound plasminogen, properties serving to localize physiologic fibrinolysis and also forming the theoretical basis for the relative fibrin selectivity of t-PA as a therapeutic agent. Soluble fibrin also stimulates activation of plasminogen by t-PA and this observation has been exploited as a potential method of measuring soluble fibrin. Thus we have demonstrated preferential degradation of soluble crosslinked fibrin dimer compared to monomer (Figure 3), consistent with the fibrin specificity of t-PA. Although the relative affinity of t-PA for solid fibrin compared to soluble crosslinked fibrin polymers is not known, it appears that therapeutic administration of t-PA would result in degradation of crosslinked fibrin polymers in preference to fibrinogen. Crosslinked fibrin polymers would be the initial component of plasma "fibrinogen" to be degraded during development of the systemic lytic state after t-PA administration.

The degradation of soluble crosslinked fibrin polymers has to be considered in evaluating results of assays for "fibrin-specific" degradation products, particularly in the setting of fibrinolytic therapy. While the monoclonal antibody directed against the γ chain crosslink epitope appears to have little reactivity with this site in soluble crosslinked fibrin, plasmic degradation of the fibrin increases immunoreactivity markedly (Figure 4). With administration of a fibrinolytic agent such as streptokinase, marked degradation of plasma fibrinogen occurs. A concomitant degradation of plasma crosslinked fibrin polymer probably occurs with a resultant elevation in plasma D-dimer. The plasma fibrinogen also decreases with administration of fibrin selective agents such as t-PA and, since plasma crosslinked fibrin polymers degrade preferentially over fibrinogen (Figure 3), an increase in plasma D-dimer would be expected in this circumstance as well. Administration of t-PA to normals has been reported to increase plasma D-dimer levels to 980 ng/ml as measured by an ELISA using antibodies different from those employed herein (Declerck, P. et al., Thromb. Haemost 58:231, 1987). After thrombolytic therapy for acute myocardial infarction, a wide range of D-dimer concentrations from 1500 ng/ml to 10,732 ng/ml has been reported using various techniques. The data presented herein (Figure 4) suggest that a large part of these elevations could derive from degradation of soluble crosslinked fibrin polymer. In contrast, the higher levels of 24,200 ng/ml and 64,000 ng/ml measures after fibrinolytic therapy for deep vein thrombosis and up to 100,000 ng/ml for pulmonary embolism could not originate entirely from plasma soluble fibrin polymers and would require additional crosslinked fibrin degradation products from lysis of thrombus.

Thus, the plasma concentration of D-dimer following t-PA degradation in vitro can be used as an indirect measure of circulating crosslinked fibrin polymer. Fragment DD with Mᵣ 195,000 represents 30% of a fibrin dimer with Mᵣ 680,000. In patients presented with acute myocardial infarction we have found using an electrophoretic technique a plasma concentration of fibrin dimer of 3.6-4.0% compared to 0.8% in normals. This observation of 3,560 ng/ml D-dimer after t-PA degradation of plasma in vitro (Figure 4) would require an initial 11,867 ng/ml of crosslinked fibrin dimer, an amount that could be provided by degradation of plasma fibrin polymer representing only 0.4% of total plasma fibrinogen (3 mg/ml), compatible with prior measurements in normals. Similarly, after thrombin exposure in vitro the value of 18,580 ng/ml (Figure 4) would derive from 61,900 ng/ml plasma crosslinked fibrin, representing 2.1% of plasma fibrinogen. These values make it clear that an in vitro degradation to D-dimer can be used as a simple indirect measure of plasma crosslinked fibrin polymer concentration as described hereinbefore.

It is to be understood that a variety of plasminogen activators may be used in this invention to generate D-dimer products. In general, any proteolytic enzyme such as streptokinase, urokinase, trypsin, etc. may be used. As indicated earlier t-PA and plasmin are preferred.

It is also to be understood that the present invention is not limited to monoclonal antibodies in the immunoassay. Thus, any antibody that is specific for D-dimer rather than for fibrinogen or fibrin may be used. As stated earlier, an appropriate monoclonal antibody is preferred however. With respect to the kit claims hereinafter it is known that immunoassay kits are of two types, the first being a direct assay which employs the sandwich approach. These kits contain a set of standards, a first antibody (i.e. capture antibody) usually immobilized on a surface and a second antibody labeled with a signal generator. The label can be any detectable entity such as radioactive, an enzyme, fluorophore, chemiphore, etc. The second type of immunoassay is the competitive type. These kits contain standards, labeled antigen and specific antibody. The kits may also contain specific buffers, substrates, separating agents and controls. The kits may contain collection devices or chemicals to treat the patients sample listed hereinbefore. In this invention the specimen could be plasma and could be drawn by conventional techniques. This kit would contain a plasminogen activator or active plasmin to treat the specimen (sample).

## Claims

1. A process for the in vitro determination of crosslinked soluble fibrin polymers in a patient sample which comprises contacting said sample with a proteolytic enzyme to generate the D-dimer fragment of said fibrin polymers and measuring the amount of said D-dimer.

2. The process according to claim 1 in which the proteolytic enzyme is tissue plasminogen activator or plasmin.

3. A process according to claim 1 in which measurement of the amount of D-dimer is carried out by the use of an immunoassay specific to said D-dimer.

4. The process according to claim 3 in which the proteolytic enzyme is tissue plasminogen activator or plasmin.

5. The process according to claims 2 or 4 in which the plasmin is produced by conversion of plasminogen to plasmin by a plasminogen activator.

6. The process according to claim 5 in which the plasminogen activator is tissue plasminogen activator.

7. The process according to claim 3 in which an antibody that is reactive with D-dimer preferentially over fibrin polymers is used in said immunoassay.

8. The process according to claim 3 in which a monoclonal antibody is used in said immunoassay.

9. The process according to claim 8 in which the monoclonal antibody exhibits the same immunological properties as those of that monoclonal antibody which is secreted by hybridoma DD/3B6/22.

10. A diagnostic kit for assaying the amount of D-dimer in a patient sample according to the process of claim 1 comprising a carrier being compartmented to receive a series of containers in close confinement which comprises:
(a) a first container containing a measured amount of standard containing crosslinked soluble fibrin polymers,
(b) a second container containing a measured amount of a proteolytic enzyme capable of generating the D-dimer fragment of said fibrin polymers, and
(c) a third container containing an antibody specific to said D-Dimer.

11. A kit according to claim 10 in which the antibody is a monoclonal antibody.

12. A kit according to claim 10 in which the monoclonal antibody exhibits the same immunological properties as those of that monoclonal antibody which is secreted by hybridoma DD/3B6/22.

13. A kit according to claim 10 in which the proteolytic enzyme is tissue plasminogen activator or plasmin.

## Patentansprüche

1. Verfahren für die in vitro-Bestimmung von vernetzten löslichen Fibrinpolymeren in einer Patientenprobe, umfassend Inberührungbringen der Probe mit einem proteolytischen Enzym für die Erzeugung des D-Dimerfragments der Fibrinpolymere und Messen der Menge des D-Dimeren.

2. Verfahren nach Anspruch 1, wobei das proteolytische Enzym Gewebe-Plasminogenaktivator oder Plasmin ist.

3. Verfahren nach Anspruch 1, wobei die Messung der Menge des D-Dimeren unter Verwendung eines für das D-Dimere spezifischen Immunassays durchgeführt wird.

4. Verfahren nach Anspruch 3, wobei das proteolytische Enzym Gewebe-Plasminogenaktivator oder Plasmin ist.

5. Verfahren nach den Ansprüchen 2 oder 4, wobei das Plasmin durch Umwandlung von Plasminogen in Plasmin mit Hilfe eines Plasminogenaktivators erzeugt wird.

6. Verfahren nach Anspruch 5, wobei der Plasminogenaktivator Gewebe-Plasminogenaktivator ist.

7. Verfahren nach Anspruch 3, wobei ein Antikörper, der mit dem D-Dimeren vorzugsweise über Fibrinpolymere reagieren kann, in dem Immunassay verwendet wird.

8. Verfahren nach Anspruch 3, wobei ein monoklonaler Antikörper in dem Immunassay verwendet wird.

9. Verfahren nach Anspruch 8, wobei der monoklonale Antikörper die gleichen immunologischen Eigenschaften wie diejenigen des monoklonalen Antikörpers zeigt, welcher durch das Hybridom DD/3B6/22 ausgeschieden wird.

10. Diagnosekit zum Untersuchen der Menge des D-Dimeren in einer Patientenprobe nach dem Verfahren des Anspruchs 1, umfassend einen Träger, welcher für die Aufnahme einer Serie von Behältern in enger Begrenzung in Kompartments unterteilt ist, umfassend:
(a) einen ersten Behälter, der eine gemessene Menge eines Standards enthält, welcher vernetzte lösliche Fibrinpolymere enthält;
(b) einen zweiten Behälter, welcher eine gemessene Menge eines proteolytischen Enzyms enthält, welches sich für die Erzeugung des D-Dimerfragments der Fibrinpolymeren eignet; und
(c) einen dritten Behälter, der einen im Hinblick auf das D-Dimere spezifischen Antikörper enthält.

11. Kit nach Anspruch 10, wobei der Antikörper ein monoklonaler Antikörper ist.

12. Kit nach Anspruch 10, wobei der monoklonale Antikörper die gleichen immunologischen Eigenschaften wie diejenigen des monoklonalen Antikörpers zeigt, welcher durch das Hybridom DD-3B6/22 ausgeschieden wird.

13. Kit nach Anspruch 10, wobei das proteolytische Enzym Gewebe-Plasminogenaktivator oder Plasmin ist.

## Revendications

1. Procédé pour doser in vitro les polymères de fibrine soluble réticulée dans l'échantillon d'un patient, qui consiste à mettre en contact l'échantillon avec une enzyme protéolytique pour produire le fragment dimère D des polymères de fibrine, et à mesurer la quantité du dimère D.

2. Procédé selon la revendication 1, dans lequel l'enzyme protéolytique est l'activateur du plasminogène tissulaire ou la plasmine.

3. Procédé selon la revendication 1, dans lequel la mesure de la quantité du dimère D est effectuée par utilisation d'une analyse immunologique spécifique du dimère D.

4. Procédé selon la revendication 3, dans lequel l'enzyme protéolytique est l'activateur du plasminogène tissulaire ou la plasmine.

5. Procédé selon la revendication 2 ou 4, dans lequel la plasmine est produite par conversion du plasminogène en plasmine par un activateur du plasminogène.

6. Procédé selon la revendication 5, dans lequel l'activateur du plasminogène est l'activateur du plasminogène tissulaire.

7. Procédé selon la revendication 3, dans lequel on utilise dans l'analyse immunologique un anticorps qui est réactif avec le dimère D, de préférence aux polymères de fibrine.

8. Procédé selon la revendication 3, dans lequel on utilise, dans l'analyse immunologique, un anticorps monoclonal.

9. Procédé selon la revendication 8, dans lequel l'anticorps monoclonal présente les mêmes propriétés immunologiques que celles de l'anticorps monoclonal qui est secrété par l'hybridome DD/3B6/22.

10. Trousse de diagnostic pour déterminer la quantité du dimère D dans l'échantillon d'un patient selon le procédé de la revendication 1, comprenant un support, compartimenté de façon à recevoir une série de récipients étroitement rapprochés, qui comprend :
(a) un premier récipient contenant une quantité dosée d'un étalon contenant les polymères de fibrine soluble réticulée,
(b) un deuxième récipient contenant une quantité dosée d'une enzyme protéolytique susceptible de produire le fragment dimère D des polymères de fibrine, et
(c) un troisième récipient contenant un anticorps spécifique du dimère D.

11. Trousse selon la revendication 10, dans laquelle l'anticorps est un anticorps monoclonal.

12. Trousse selon la revendication 10, dans laquelle l'anticorps monoclonal présente les mêmes propriétés immunologiques que celles de l'anticorps monoclonal qui est secrété par l'hybridome DD/3B6/22.

13. Trousse selon la revendication 10, dans laquelle l'enzyme protéolytique est l'activateur du plasminogène tissulaire ou la plasmine.
